# EUROPEAN PATENT SPECIFICATION

(11) **EP 3 094 303 B1**
(45) Date of publication and mention of the grant of the patent: **16.01.2019**
(21) Application number: 13789706.2
(22) Date of filing: 29.10.2013
(51) Int. Cl.: A61K 8/9789, A61Q 19/00, A61Q 19/08, A61K 8/92, A61K 8/31, A61K 8/34, A61K 8/49, A61K 8/65

(54) **COMPOSITION FOR THE EFFECTS OF WINTER AGING ON THE SKIN**
ZUSAMMENSETZUNG GEGEN DIE AUSWIRKUNGEN VON WINTERALTERUNG AUF DIE HAUT
COMPOSITION POUR LES EFFETS DU VIEILLISSEMENT HIVERNAL SUR LA PEAU

(43) Date of publication of application: 23.11.2016
(73) Proprietor: Taylor, Robert Peter, Little Venice London W9 1AH (GB); Lalvani, Kartar Singh, London NW2 7HF (GB); Lalvani, Ajit, Oxford OX2 7ES (GB)
(72) Inventor: Taylor, Robert Peter, Little Venice London W9 1AH (GB); Lalvani, Kartar Singh, London NW2 7HF (GB); Lalvani, Ajit, Oxford OX2 7ES (GB)
(86) International application number: PCT/EP2013/003247
(87) International publication number: WO 2015/062615

(56) References cited:
- WO-A1-2013/054304
- WO-A2-2012/120290
- GB-A- 2 458 466
- US-A1- 2011 052 511
- US-A1- 2011 281 941

## Description

### TECHNICAL FIELD

This invention concerns a composition of natural occurring constituents in tablet or capsule form for use in combating the damaging effects of winter aging on the skin.

### BACKGROUND ART

Skin is made up of three layers : the epidermis, the dermis and subcutaneous fat. The epidermis, which is the topmost, outer layer is somewhat translucent, allowing light to partially pass through it. The thickness of the epidermis varies in different types of skin. There are no blood vessels in the epidermis. Hence this top layer gets its nutrients and oxygen from the deeper layers of the skin. The dermis is composed of three types of tissues, namely collagen, elastic tissue, and reticular fibres. The sweat glands and hair follicles also reside in this layer along with some blood vessels and nerves. The subcutaneous layer primarily provides insulation and padding and thins as it ages. The depth of the subcutaneous fat layer differs from one person to another. This layer is attached to the bones and muscles by connective tissue, thereby allowing the skin to move. It is the upper levels of the epidermis and dermis that are most under attack from the elements. It is also essential to protect the subcutaneous fat in order to preserve a more flawless and wrinkle-free complexion.

For decades it has been appreciated that aging of the skin is the consequence of both genetic and environmental influences. Among all environmental factors, solar ultraviolet (UV) radiation during the summer is most important for extrinsic skin aging, a process accordingly also termed photo aging. Vitamins and micronutrients have been used in systemic and topical photoprotection. Dietary photoprotection through the administration of carotenoids, tocopherol and vitamin C in foods or supplements has been successfully used to prevent UV-induced erythema.

Among the extrinsic causes, UVB and also UVA, play a preponderant role. The phenomena of aging provoke the decline in defence, healing and perception mechanisms and in the thermoregulation of the skin tissue. There are numerous and often unsightly clinical manifestations. Photo-aging can be considered as a marker of risk of photo-carcinogenesis requiring increased clinical surveillance.

However, even in normal adults, the dry and cold winter season also has a great influence on the condition of the skin, and especially affects exposed areas such as the face more than genrally covered areas such as the forearm. Clinical observation revealed that approximately one-third of children exhibited visible dry skin changes in winter. Although the stratum corneum hydration and barrier function may behave independently in some skin conditions, such as senile xerosis and fresh scars, they show concomitant changes in scaly inflammatory skin lesions. Even individuals with normal skin show subclinical changes on their facial skin when exposed to the dryness and coldness of winter, which can be demonstrated biophysically as decreased stratum corneum hydration and deteriorated stratum corneum barrier function. There is some differences regarding the skin elasticity among different seasons. It was indicated that the most extreme seasons in Shanghai, which are summer and winter, induce changes in skin. The latter, however, concern functional rather than structural properties. Wrinkling and sagging, two macroscopic facets of aging skin, did not show any seasonal influence. Such findings are not surprising for two major reasons. Firstly, skin aging is a rather slow process, more easily evidenced over years and decades than months. Secondly, structural changes such as sagging and wrinkling clearly originate in alterations in the dermis, which is a tissue with a slow turnover. Therefore, these microscopic changes, year after year, may lead to the macroscopic and visible skin aging signs.

The female is more susceptible to environmental factors for two different reasons: (a) the female skin thickness is significantly less than the male and (b) the tissue dielectric constant (TDC) is 13% higher in male skin than that of females. This parameter is a reliable marker for local tissue water. All of these evidences suggest : (1) the skin dryness increases during winter time, (2) the exposed areas are more susceptible to the seasonal changes and (3) the females' skin is more prone to these changes.

Several studies have confirmed the dramatic changes of skin surface parameters during winter but there are no published studies demonstrating the effects of an oral supplementation on preventing the negative winter effects on the skin. Therefore, the purpose of the present invention is to prevent and correct the effects of a negative seasonal change of the skin surface and deep parameters by using a specific multi-nutrient supplement.

The inventors previously devised a formulation for use with skin in order to help to diminish the negative effects of UV radiation and to promote skin radiance Reference is made to GB2458466.

The constituents included in that formulation were : Bio-Marine Collagen (Cartidea), Grape Seed Extract (95% Proanthocyanidins), Pine Bark Extract (95% Proanthocyanidins), Green Tea Extract (Standardised extract 55% polyphenols), Lycopene (Extract 5%), Blackcurrant Seed Oil, Alpha Lipoic Acid, Co-enzyme Q10, Vitamin A (Betacarotene), Vitamin D (Vitamin D3), Vitamin E (Natural Source), Vitamin C, Vitamin B1 (Thiamin), Vitamin B2 (Riboflavin), Vitamin B3 (Niacin), Vitamin B6 (Pyridoxine), Folic Acid, Vitamin B12, Biotin, Vitamin B5 (Pantothenic Acid), Magnesium, Iron, Zinc, Copper, Manganese, Selenium (Yeast Free), Chromium, Iodine (from Purified Sea Kelp extract), Silicon, L-Cystine. The constituents were chosen because of their potential involvement in the biochemical functions of the skin.

### DISCLOSURE OF INVENTION

Summer skin aging is well known but winter skin aging, which is not caused by UV radiation or other common causes, is a new concept and use for the formulation that was used previously. The purpose of the present invention is the use of the previous formulation for use with winter skin aging. Winter skin aging is a new use for that formulation and thereby constitutes a second medical use. However, it was not known whether or not the biochemical constituents used previously for other effects on the skin would also combat the effects on skin that occur during the winter.

### Clinical trial

In order to assess the possible use and efficacy of the previous formulation in winter skin aging the applicants carried out an entirely confidential double blind placebo controlled clinical trial.

### Objective

The purpose was to evaluate the efficacy of the oral micronutrient supplementation on preventing the negative effects of winter time on the skin quality by using non-invasive biometrologic instruments.

### Subjects

This study was a monocentric, randomised, double blinded, placebo controlled comparative study featuring 80 healthy female volunteers, aged 35 to 55 years old with phototype II-IV. All subjects had significant photo aging of the face based on the photographic scale without sun and UV exposition 48 hours before the first day and during the whole study. Subjects accepted using only basic moisturising products for daily skin care. They were not participating in any other research during the study. The study was conducted in the winter time (from October to April) in France with the local mean temperature of -4°C in January and +18.7°C in April.

Exclusion criteria were pregnancy or lactation, using oral nutritional complements and/or vitamin supplementation two months before the study, using cosmetics containing ingredients with anti-aging purposes (e.g. vitamin C, vitamin A, retinaldehyde or similar), hypersensibility to one or several ingredient(s) of the study product, any acute or chronic serious disease, any skin disease, and any systemic or local treatment such as topical or oral corticosteroids, diuretics etc. possibly interfering with the measured parameters.

### Methods

Two tablets of the micronutrient supplement was administered once daily during the main meal for 4 months. The volunteers were examined at T0 (before treatment), T4 months (122 days, end of treatment) and at T5.5 months (6 weeks after treatment termination). Randomisation was balanced according to 2 blocks and 8 layers concerning the age group, skin dryness and hormone replacement therapy.

All of the subjects were recommended to wash their face and arms on the evening preceding the examination days (T0, T4 months and T5.5 months) and then not to apply anything on their face and arms (including no water, no soap, shower gel, cream, or make-up) up until the point of the examination.

The measurements took place in a temperature (20±2° C) and humidity (55±5%) regulated environment. The volunteers stayed quiet for at least 15 minutes in stable environmental conditions. The skin was not subject to any strain or stress. All measures were performed in the supine position. Measurement areas were the face using digital photography and skin relief, and the anterior side of the forearm (as non-exposed zone) and back of the hand (as exposed zone) : skin visco-elasticity and high frequency ultrasound imaging. The measurement areas were recorded by using a flexible transparent plastic mask. This allowed measuring exactly at the same place at each visit (Thermocell-Test professional Kit).

Standardised photographs of the face were taken with a Nikon D70 digital camera with objective NIKON AF Micro-Nikkor 60 mm. The photographs were taken in sitting position, under normal and polarised light for the face, front and profile with same positioning for the camera, the same light conditions and the same distance to the skin (1 m) for each visit (stereotaxic facial system including a metallic platform). The photo evaluation was performed by a panel of experienced professionals according to the 6 grade photographic scaling used to grade the overall severity of facial photodamage as Grade 1 to 6 (mild, mild / moderate, moderate, moderate / severe, severe, very severe).

Skin relief was evaluated by Visioscan VC 98. The Visioscan VC 98 features a black-and-white high-performance digital camera taking pictures of the skin surface under standardised homogenous ring-shaped UV A-illumination (340-400 nm, peak 375 nm). Each picture of 640 x 480 pixels was analysed by the dedicated software (Visioscan 2000) and parameters representing pseudo-roughness [R1(Rt), R2(Rm), R3(Rz), wrinkles deep indicators)] and micro relief [R4(Rp), R5(Ra), micro-relief indicators].

Rz or circular roughness is the arithmetic average of five measurements of the distance between the highest and the lowest value, referred to a reference length 1, on a selected line. Lines can be arranged horizontally, vertically, or circularly. The advantage of circularly arranged lines is that any influence from the direction of the wrinkles is compensated.

The SELS (Surface Evaluation of the Living Skin) method is based on a graphic depiction of the living skin under special illumination. The electronic processing and evaluation of this image was carried out according to two clinical parameters : Skin roughness (Ser) is the roughness parameter and calculates the grey levels above the threshold in comparison with the entire image (Reflects the 'asperity' of the skin), Wrinkles (Sew) is calculated from the proportion of horizontal and vertical wrinkles (Identifies aging including wrinkles).

In order to measure the skin viscoelasticity a sensitive biometrological device, Reviscometer RVM 600 17, was used. It measures the propagation velocity of acoustic shock waves. For this purpose, two sensors are applied on the skin surface. The mean value of the RRT parameter (Resonance Running Time) over these 4 axes is calculated, as well as the values at 0-180° and 90-270° separately. Four measurements were conducted on each zone (inner forearm and back of the hand) in each 4 directions and mean values were calculated.

An ultrasonic wave (frequency 20 MHz) was applied via Echographic device (Dermcup) on the surface of the skin by means of a suitable probehead. Three images were taken on each measurement zone (inner forearm and back of the hand). For each image, three measurements of skin thickness were conducted (3 values/zone/volunteer) and mean values were calculated. The data were analysed by proprietary software (Skinexigence). The thickness is calculated from a Region of Interest (ROI) determined directly on the image. The density is defined as the mean ROI luminance.

The subjects answered the self-evaluation questionnaire on visit of 4th month. The statistical analysis was conducted with the programs Statistica 6.0 and Instat GraphPad 3.0. The analyses were based on the Intend-To-Treat (ITT) population which comprised all subjects included in the study. Statistical significance was set at 5% (double sided) and at 5% to 10 % for trends.

Descriptive statistics were reported for each parameter: mean standard deviation, minimum, maximum, and median, quartiles for quantitative data and percentages for qualitative data.

Quantitative variables underwent an Analysis of Variance (ANOVA; if normal distribution was ensured or Friedman test; if not normally distributed). With these tests, the effect of time (T0, T4 months and T 5.5 months) was investigated. If the time effect was statistically significant, the test was completed by a test for multiple comparisons (called Dunn's test).

### Results

The main criterion was the evaluation of cutaneous fine lines, skin micro-relief and wrinkles on the face. The secondary criteria were skin thickness and density, the subject's self-assessment, and the illustrative macrophotography and tolerance.

Eighty (80) healthy Caucasian female volunteers aged 36 to 55 years (mean = 46 ± 6 years, mean active group 46.6, mean placebo group 46.4) were enrolled. One subject terminated the study prematurely because of an intolerance reaction to the placebo tablet (results in the placebo group concerned with n = 39 subjects). Mean Body mass index (BMI) in the study population was in normal weight range (20-25) (Mean BMI in active group = 22.7± 2.1 and in placebo group = 23.4 ± 4.3). 30% of the active group and 35% of the placebo group were post menopausal. In the active group, 3 subjects (7.5%) were undergoing hormone replacement therapy for 2y, 8y and 4 months while 3 subjects (7.65%) were undergoing hormone replacement therapy for 3y, 5y and 16y in the placebo group. 43% of active and 45% of placebo group had skin dryness according the subject assertion. No differences were observed in terms of smoking habit between both groups (7.5±5.6 pack/year in active and 7.5±5.1 pack/year in placebo group). No serious side effects were observed.

Skin Relief was the main criterion. For all pseudo-roughness and micro-relief indicators like Rt (R1), Rm (R2), Rz (R3) and Ra, there was a significant negative increase from T0 to T4 in placebo group [for Rt (R1) between T4 and T5.5 (p<0.01), Rm (R2) between T4 and T5.5 (p<0.01) and for Rz (R3) between T0 and T5.5 (p<0.05)] while there was no adverse change in the active group. Considering the seasonal time of this study, this difference can be considered as a protective effect of the active supplement against skin dryness during winter. This effect is also observable for Rp (R5) but it is not statistically significant. No significant effects were found for SELS (Surface Evaluation of Living Skin) parameters (Ser and Sew).

Secondary criteria was Viscoelasticity. No difference was observed in mean RRT values (multidirectional) between two groups in non-exposed (forearm) and also exposed area (dorsum of the hand). On the non-exposed zone (forearm), the most important differences were observed on 45° and 135° axis. Data showed that the Resonance Running Time at 45° (RRT, millisecond) was significantly decreased in active group by 13% from T0 to T5.5 (p< 0.05) (Table 4). Descriptive statistics of the mean minimum, mean maximum and Min/Max ratio showed a non-significant difference between T0 and T4 (P=0.0579) and between T0 and T5.5 (p=0.0512) in the active group. On the exposed zone (dorsum of the hand), the most significant differences were observed in 90° between T0 and T5.5 in active group. The non-parametric Friedmann test did not show any time effect for mean RRT neither in the active nor the placebo group while the Dunn's test pointed out a significant difference between T0 and T5.5 in both active (p < 0.01) and placebo group (p < 0.01).

Descriptive statistics of the mean minimum, mean maximum and Min/Max ratio showed a significant dramatic difference between T0 and T4 (P=0.0001) and between T0 and T5.5 (p=0.0008) in the active group, which was not significant in placebo group (p=0.072).

On unexposed study zone (forearm), the skin thickness and density was significantly increased in both group (p< 0.05) but more important changes between T0 and T4 were observed in the active group (non-significantNS).

In the active group, the skin thickness was decreased after stopping supplementation (p<0.05) but no changes were observed for placebo group (p>0.05). The skin density continued to increase in both groups between T4 and T5.5 but was more pronounced in the active group (non-significant). In contrast, on exposed study zone (back of hand), the skin thickness was decreased significantly in placebo group between T0 and T4 (winter time) (p < 0.01, Dunn's test) which was not significant in the active groups. The skin density was improved significantly in both group (p< 0.05) which was not treatment related.

Photographic evaluation - The mean grade evaluation for active group was 3.0 and for placebo group was 3.1 at T0 which did not change during the study.

Subjects self-assessments - The self assessment questionnaire revealed positive differences in increasing elasticity and firmness and also decreasing roughness and dryness in active group however this did not reach statistical significance.

### Discussion

The previous studies revealed that the winter seasonal changes could have negative effects on the skins structural components e.g. free fatty acids, linoleic acid, some saturated fatty acids, oleic acid, SC lipids, Ceramide 1 linoleate, ceramide 1 Oleate and biometrologic parameters including skin water content, TEWL and Skin sebum. On the other hand, low humidity can stimulate the epidermal DNA synthesis as a hyperproliferative reaction to barrier disruption. There is limited information to prove the skin elasticity changes during winter time. This shows that there is a necessity to provide an intervention to prevent the negative effects of winter time. There are many studies confirming the positive effects of the topical treatments, but no published study for preventive effects of an oral supplementation which can be effective on exposed areas to the winter elements.

The study protocol was the same as other studies in this field but the panel size (80 subjects) was larger and for the first time the study was designed to investigate protective effects of oral supplementation over the winter time. As observed in the previous studies, the macroscopic clinical signs of wrinkles and firmness did not show particular change over the 6 months period because facial wrinkle formation and sagging are long term cumulative effects.

It was observed that one of the best parameters for studying the skin surface changes among the skin relief parameters is circular roughness but in the present study all other pseudo-roughness indicators like Rt and Rm also showed a significant augmentation in placebo group during winter time which can describe the protective effects of the nutritional supplementations in active group (p<0.05). Considering the seasonal time, this difference can be considered as a protective effect of the active supplement against skin dryness during winter. These results were confirmed by the micro-relief indicators (Ra and Rp) as well. No significant effects were pointed out for SELS (Surface Evaluation of Living Skin) parameters (Ser and Sew).

The results obtained for skin thickness and density by high frequency ultrasound revealed interesting effects of environmental factors in winter. The skin thickness of non-exposed area (forearm) did not show any difference between groups while the skin thickness of exposed area (dorsum of hand) was decreased significantly in placebo group between T0 and T4 (winter time) (p<0.01, Dunn's test) which was not significant in the active group. This result confirmed the protective effects of the nutritional supplement on skin thickness in exposed areas. On the other hand, the results showed the skin thickness was decreased after stopping supplementation (p<0.05). These findings highlight the importance of continuing the treatment longer than the duration of this particular study. Defining the minimum treatment duration needs further longer studies.

The photographic scale has a limited benefit for this type of study because the clinical signs of the skin aging including facial wrinkles and sagging cannot considerably be changed during such a short period of time. However the protection of the skin structure observed, if repeated over a number of winter periods could lead to long term macroscopic clinical improvement.

### Conclusion

The results observed in this clinical trial indicate that the skin is prone to the seasonal changes in winter, particularly in exposed areas. The clinical data also supports the contention that supplementation with the formulation used in the clinical trial can be a safe treatment and can prevent or even eliminate the negative aging effects that winter has on the skin.

### Formulation

The following are the active constituents of the optimal composition. The optimal forms but not the only possible forms are in brackets :
Bio-Marine Collagen (Cartidea)
Grape Seed Extract (95% Proanthocyanidins)
Pine Bark Extract (95% Proanthocyanidins)
Green Tea Extract (Standardised extract 55% polyphenols)
Lycopene (Extract 5%)
Blackcurrant Seed Oil
Alpha Lipoic Acid
Co-enzyme Q10
Vitamin A (Betacarotene)
Vitamin D (Vitamin D3)
Vitamin E (Natural Source)
Vitamin C
Vitamin B1 (Thiamin)
Vitamin B2 (Riboflavin)
Vitamin B3 (Niacin)
Vitamin B6 (Pyridoxine)
Folic Acid
Vitamin B12
Biotin
Vitamin B5 (Pantothenic Acid)
Magnesium
Iron
Zinc
Copper
Manganese
Selenium (Yeast Free)
Chromium
Iodine (from Purified Sea Kelp extract)
Silicon
L-Cystine

More concise but lesser forms of the composition can comprise :
(a) Bio-Marine Collagen, Grape Seed Extract, Pine Bark Extract, Green Tea Extract, Lycopene, Blackcurrant Seed Oil, Alpha Lipoic Acid, Co-enzyme Q10.
(b) Bio-Marine Collagen, Grape Seed Extract, Pine Bark Extract, Green Tea Extract, Lycopene, Blackcurrant Seed Oil, Alpha Lipoic Acid, Co-enzyme Q10. Vitamin C, Vitamin E, Folic Acid, Vitamin B12, Iron, Zinc, Iodine.
(c) Bio-Marine Collagen, Grape Seed Extract, Pine Bark Extract, Green Tea Extract, Lycopene, Blackcurrant Seed Oil, Alpha Lipoic Acid, Co-enzyme Q10. Vitamin C, Vitamin E, Folic Acid, Vitamin B12, Iron, Zinc, Iodine, Vitamin A, Vitamin B1, Vitamin B2, Vitamin B3, Selenium, Chromium, Silicon.

More specific uses are the improvement of the viscoelasticity of the skin, increasing skin thickness, protection against roughness of the skin, and protection against increases in skin micro relief.

The following are the optimal dosages of the optimal composition. It is preferable to consume the formulation in two tablets or to a lesser extent in capsules. The preferred ranges are in brackets. Other dosages can be used other than those stated. However, the further the dosages are from the optimal dosages the less effective the composition is likely to be :
Bio-Marine Collagen (Cartidea) - 200 mg (100 mg to 400 mg)
Grape Seed Extract (95% Proanthocyanidins) - 15 mg (10 mg to 30 mg)
Pine Bark Extract (95% Proanthocyanidins) - 8 mg (4 mg to 16 mg)
Green Tea Extract (Standardised extract 55% polyphenols) - 20 mg (10 mg to 40 mg)
Lycopene (Extract 5%) - 10 mg (5 mg to 20 mg)
Blackcurrant Seed Oil - 50 mg (25 mg to 100 mg)
Alpha Lipoic Acid - 40 mg (20 mg to 80 mg)
Co-enzyme Q10 - 5 mg (2.5 mg to 10 mg)
Vitamin A (Betacarotene) - 4 mg (2 mg to 8 mg)
Vitamin D (Vitamin D3) - 12.5 mcg (500 IU) (6 mcg to 25 mcg)
Vitamin E (Natural Source) - 50 mg (25 mg to 100 mg)
Vitamin C - 90 mg (45 mg to 180 mg)
Vitamin B1 (Thiamin) - 8 mg (2 mg to 16 mg)
Vitamin B2 (Riboflavin) - 4 mg (2 mg to 8 mg)
Vitamin B3 (Niacin) - 18 mg (9 mg to 36 mg)
Vitamin B6 (Pyridoxine) - 10 mg (2mg to 20 mg)
Folic Acid - 400 mcg (200 mcg to 800 mcg)
Vitamin B12 - 9 mcg (5 mcg to 18 mcg)
Biotin- 150 mcg (75 mcg to 300 mcg)
Vitamin B5 (Pantothenic Acid) - 40 mg (20mg to 80 mg)
Magnesium - 100 mg (50 mg to 200mg)
Iron - 12 mg (6 mg to 24 mg)
Zinc - 15 mg (7.5 mg to 30 mg)
Copper - 1 mg (0.5mg to 2 mg)
Manganese - 0.5 mg (0.25 mg to 1 mg)
Selenium (Yeast Free) - 100 mcg (50 mcg to 200 mcg)
Chromium - 50 mcg (25 mcg to 100 mcg)
Iodine (from Purified Sea Kelp extract) - 200 mcg (100 mcg to 400 mcg)
Silicon - 50 mg (25 mg to 100 mg)
L-Cystine - 20 mg (10 mg to 40 mg)

### INDUSTRIAL APPLICABILITY

According to this invention, there is a composition for protection against the aging effects of winter on the skin in which the active constituents of the composition are : Bio-Marine Collagen (Cartidea), Grape Seed Extract (95% Proanthocyanidins), Pine Bark Extract (95% Proanthocyanidins), Green Tea Extract (Standardised extract 55% polyphenols), Lycopene Extract 5%, Blackcurrant Seed Oil, Alpha Lipoic Acid, Co-enzyme Q10, Betacarotene, Vitamin D (D3 500 IU), Vitamin E (Natural Source), Vitamin C, Vitamin B1 (Thiamin), Vitamin B2 (Riboflavin), Vitamin B3 (Niacin), Vitamin B6, Folic Acid, Vitamin B12, Biotin, Pantothenic Acid, Magnesium, Iron, Zinc, Copper, Manganese, Selenium (Yeast Free), Chromium, Iodine (from Purified Sea Kelp extract), Silicon, L-Cystine.

## Claims

1. Use of a composition comprising : Bio-Marine Collagen, Grape Seed Extract, Pine Bark Extract, Green Tea Extract, Lycopene, Blackcurrant Seed Oil, Alpha Lipoic Acid, Co-enzyme Q10, Vitamin C, Vitamin E, Folic Acid, Vitamin B12, Iron, Zinc, Iodine, in the protection against the effects of winter in the skin.

2. Use according to Claim 1 **characterized in that** the composition also comprises : Betacarotene, Vitamin B1, Vitamin B2, Vitamin B3, Selenium, Chromium, Silicon.

3. Use according to Claim 2 **characterized in that** the composition also comprises : Vitamin D, Vitamin B6, Biotin, Pantothenic Acid, Magnesium, Copper, Manganese, L-Cystine.

4. Use according to Claim 3 **characterized in that** the following forms of the substances are used : Bio-Marine Collagen (Cartidea), Grape Seed Extract (95% Proanthocyanidins), Pine Bark Extract (95% Proanthocyanidins), Green Tea Extract (Standardised extract 55% polyphenols), Lycopene Extract 5%, Blackcurrant Seed Oil, Alpha Lipoic Acid, Co-enzyme Q10, Betacarotene, Vitamin D (D3 500 IU), Vitamin E (Natural Source), Vitamin C, Vitamin B1 (Thiamin), Vitamin B2 (Riboflavin), Vitamin B3 (Niacin), Vitamin B6, Folic Acid, Vitamin B12, Biotin, Pantothenic Acid, Magnesium, Iron, Zinc, Copper, Manganese, Selenium (Yeast Free), Chromium, Iodine (from Purified Sea Kelp extract), Silicon, L-Cystine.

5. Use according to Claim 4 **characterized in that** the following range of dosages are used : 100 mg to 400 mg Bio-Marine Collagen (Cartidea), 10 mg to 30 mg Grape Seed Extract (95% Proanthocyanidins), 4 mg to 16 mg Pine Bark Extract (95% Proanthocyanidins), 10 mg to 40 mg Green Tea Extract (Standardised extract 55% polyphenols), 5 mg to 20 mg Lycopene (Extract 5%), 25 mg to 100 mg Blackcurrant Seed Oil, 20 mg to 80 mg Alpha Lipoic Acid, 2.5 mg to 10 mg Co-enzyme Q10, 2 mg to 8 mg Betacarotene, 6 mcg to 25 mcg Vitamin D3, 25 mg to 100 mg Vitamin E (Natural Source), 45 mg to 180 mg Vitamin C, 2 mg to 16 mg Thiamin, 2 mg to 8 mg Riboflavin, 9 mg to 36 mg Niacin, 2 mg to 20 mg Vitamin B6, 200 mcg to 800 mcg Folic Acid, 5 mcg to 18 mcg Vitamin B12, 75 mcg to 300 mcg Biotin, 20 mg to 80 mg Vitamin B5 (Pantothenic Acid), 50 mg to 200 mg, 6 mg to 24 mg Iron, 7.5 mg to 30 mg Zinc, 0.5mg to 2 mg Copper, 0.25 mg to 1 mg Manganese, 50 mcg to 200 mcg Selenium (Yeast Free), 25 mcg to 100 mcg Chromium, 100 mcg to 400 mcg Iodine (from Purified Sea Kelp extract), 25 mg to 100 mg Silicon, 10 mg to 40 mg L-Cystine.

6. Use according to Claim 4 **characterized in that** the following dosages are used : 200 mg Bio-Marine Collagen (Cartidea), 15 mg Grape Seed Extract (95% Proanthocyanidins), 20 mg Green Tea Extract (Standardised extract 55% polyphenols), 8mg Pine Bark Extract (95% Proanthocyanidins), 10mg Lycopene Extract 5%, 50mg Blackcurrant Seed Oil, 40mg Alpha Lipoic Acid, 5mg Co-enzyme Q10, 4 mg Betacarotene, 12.5 mcg Vitamin D (D3 500 IU), 50mg Vitamin E (Natural Source), 90mg Vitamin C, 8mg Vitamin B1 (Thiamin), 4 mg Vitamin B2 (Riboflavin), 18 mg Vitamin B3 (Niacin), 10mg Vitamin B6, 400 mcg Folic Acid, 9 mcg Vitamin B12, 150 mcg Biotin, 40mg Pantothenic Acid, 100mg Magnesium, 12 mg Iron, 15mg Zinc, 1 mg Copper, 0.5mg Manganese, 100mcg Selenium (Yeast Free), 50 mcg Chromium, 200mcg Iodine (from Purified Sea Kelp extract), 50mg Silicon, 20mg L-Cystine.

7. Use according to any one of the preceding claims in which the composition includes one or more carriers or excipients.

8. Use according to any one of the preceding claims in which the protection against the effects of winter on the skin specifically concerns : the improvement of the viscoelasticity of the skin, increasing skin thickness, protection against roughness of the skin, and protection against increases in skin micro relief.

## Patentansprüche

1. Verwendung einer Zusammensetzung, umfassend: Bio-Marine-Collagen, Traubenkernextrakt, Kiefernrindenextrakt, Grüntee-Extrakt, Lycopin, Schwarze-Johannisbeer-Samenöl, Alpha-Liponsäure, Co-Enzym Q10, Vitamin C, Vitamin E, Folsäure, Vitamin B12, Eisen, Zink, Iod, beim Schutz gegen die Auswirkungen des Winters auf die Haut.

2. Verwendung nach Anspruch 1, **dadurch gekennzeichnet, dass** die Zusammensetzung außerdem Folgendes umfasst: Beta-Carotin, Vitamin B1, Vitamin B2, Vitamin B3, Selen, Chrom, Silizium.

3. Verwendung nach Anspruch 2, **dadurch gekennzeichnet, dass** die Zusammensetzung außerdem Folgendes umfasst: Vitamin D, Vitamin B6, Biotin, Pantothensäure, Magnesium, Kupfer, Mangan, L-Cystin.

4. Verwendung nach Anspruch 3, **dadurch gekennzeichnet, dass** die folgenden Formen der Substanzen verwendet werden: Bio-Marine-Collagen (Cartidea), Traubenkernextrakt (95% Proanthocyanidine), Kiefernrindenextrakt (95% Proanthocyanidine), Grüntee-Extrakt (standardisierter Extrakt 55% Polyphenole), Lycopin-Extrakt 5%, Schwarze-Johannisbeer-Samenöl, Alpha-Liponsäure, Co-Enzym Q10, Beta-Carotin, Vitamin D (D3 500 IE), Vitamin E (natürliche Quelle), Vitamin C, Vitamin B1 (Thiamin), Vitamin B2 (Riboflavin), Vitamin B3 (Niacin), Vitamin B6, Folsäure, Vitamin B12, Biotin, Pantothensäure, Magnesium, Eisen, Zink, Kupfer, Mangan, Selen (hefefrei), Chrom, Iod (aus gereinigtem Seetang-Extrakt), Silizium, L-Cystin.

5. Verwendung nach Anspruch 4, **dadurch gekennzeichnet, dass** folgende Dosisbereiche verwendet werden: 100 mg bis 400 mg Bio-Marine-Collagen (Cartidea), 10 mg bis 30 mg Traubenkernextrakt (95% Proanthocyanidine), 4 mg bis 16 mg Kiefernrindenextrakt (95% Proanthocyanidine), 10 mg bis 40 mg Grüntee-Extrakt (standardisierter Extrakt 55% Polyphenole), 5 mg bis 20 mg Lycopin (Extrakt 5%), 25 mg bis 100 mg Schwarze-Johannisbeer-Samenöl, 20 mg bis 80 mg Alpha-Liponsäure, 2,5 mg bis 10 mg Co-Enzym Q10, 2 mg bis 8 mg Beta-Carotin, 6 µg bis 25 µg Vitamin D3, 25 mg bis 100 mg Vitamin E (natürliche Quelle), 45 mg bis 180 mg Vitamin C, 2 mg bis 16 mg Thiamin, 2 mg bis 8 mg Riboflavin, 9 mg bis 36 mg Niacin, 2 mg bis 20 mg Vitamin B6, 200 µg bis 800 µg Folsäure, 5 µg bis 18 µg Vitamin B12, 75 µg bis 300 µg Biotin, 20 mg bis 80 mg Vitamin B5 (Pantothensäure), 50 mg bis 200 mg, 6 mg bis 24 mg Eisen, 7,5 mg bis 30 mg Zink, 0,5 mg bis 2 mg Kupfer, 0,25 mg bis 1 mg Mangan, 50 pg bis 200 µg Selen (hefefrei), 25 µg bis 100 µg Chrom, 100 µg bis 400 µg Iod (aus gereinigtem Seetang-Extrakt), 25 mg bis 100 mg Silizium, 10 mg bis 40 mg L-Cystin.

6. Verwendung nach Anspruch 4, **dadurch gekennzeichnet, dass** die folgenden Dosierungen verwendet werden: 200 mg Bio-Marine-Collagen (Cartidea), 15 mg Traubenkernextrakt (95% Proanthocyanidine), 20 mg Grüntee-Extrakt (standardisierter Extrakt 55% Polyphenole), 8 mg Kiefernrindenextrakt (95% Proanthocyanidine), 10 mg Lycopin-Extrakt 5%, 50 mg Schwarze-Johannisbeer-Samenöl, 40 mg Alpha-Liponsäure, 5 mg Co-Enzym Q10, 4 mg Beta-Carotin, 12,5 µg Vitamin D (D3 500 IE), 50 mg Vitamin E (natürliche Quelle), 90 mg Vitamin C, 8 mg Vitamin B1 (Thiamin), 4 mg Vitamin B2 (Riboflavin), 18 mg Vitamin B3 (Niacin), 10 mg Vitamin B6, 400 µg Folsäure, 9 µg Vitamin B12, 150 µg Biotin, 40 mg Pantothensäure, 100 mg Magnesium, 12 mg Eisen, 15 mg Zink, 1 mg Kupfer, 0,5 mg Mangan, 100 µg Selen (hefefrei), 50 µg Chrom, 200 µg Iod (aus gereinigtem Seetang-Extrakt), 50 mg Silizium, 20 mg L-Cystin.

7. Verwendung nach einem der vorhergehenden Ansprüche, bei welcher die Zusammensetzung einen oder mehrere Träger oder Hilfsstoffe enthält.

8. Verwendung nach einem der vorhergehenden Ansprüche, bei welcher der Schutz gegen die Auswirkungen des Winters auf die Haut spezifisch Folgendes betrifft: die Verbesserung der Viskoelastizität der Haut, die Erhöhung der Hautdicke, den Schutz gegen Rauhigkeit der Haut und den Schutz gegen Erhöhungen des Haut-Mikroreliefs.

## Revendications

1. Utilisation d'une composition comprenant : du collagène bio-marin, un extrait de pépins de raisin, un extrait d'écorce de pin, un extrait de thé vert, du lycopène, de l'huile de pépins de cassis, de l'acide alpha-lipoïque, du co-enzyme Q10, de la vitamine C, de la vitamine E, de l'acide folique, de la vitamine B12, du fer, du zinc, de l'iode, dans la protection de la peau contre les effets de l'hiver.

2. Utilisation selon la revendication 1, **caractérisée en ce que** la composition comprend également : du bêta-carotène, de la vitamine B1, de la vitamine B2, de la vitamine B3, du sélénium, du chrome, du silicium.

3. Utilisation selon la revendication 2, **caractérisée en ce que** la composition comprend également : de la vitamine D, de la vitamine B6, de la biotine, de l'acide pantothénique, du magnésium, du cuivre, du manganèse, de la L-cystine.

4. Utilisation selon la revendication 3, **caractérisée en ce que** les formes suivantes des substances sont utilisées : du collagène bio-marin (Cartidea), un extrait de pépins de raisin (95% de proanthocyanidines), un extrait d'écorce de pin (95% de proanthocyanidines), un extrait de thé vert (extrait standardisé à 55% de polyphénols), un extrait de lycopène à 5%, de l'huile de pépins de cassis, de l'acide alpha-lipoïque, du co-enzyme Q10, du bêta-carotène, de la vitamine D (D3, 500 UI), de la vitamine E (source naturelle), de la vitamine C, de la vitamine B1 (thiamine), de la vitamine B2 (riboflavine), de la vitamine B3 (niacine), de la vitamine B6, de l'acide folique, de la vitamine B12, de la biotine, de l'acide pantothénique, du magnésium, du fer, du zinc, du cuivre, du manganèse, du sélénium (exempt de levure), du chrome, de l'iode (issue d'un extrait de varech purifié), du silicium, de la L-cystine.

5. Utilisation selon la revendication 4, **caractérisée en ce que** les plages suivantes de dosages sont utilisées : de 100 mg à 400 mg de collagène bio-marin (Cartidea), de 10 mg à 30 mg d'extrait de pépins de raisin (95% de proanthocyanidines), de 4 mg à 16 mg d'extrait d'écorce de pin (95% de proanthocyanidines), de 10 mg à 40 mg d'extrait de thé vert (extrait standardisé à 55% de polyphénols), de 5 mg à 20 mg de lycopène (extrait à 5%), de 25 mg à 100 mg d'huile de pépins de cassis, de 20 mg à 80 mg d'acide alpha-lipoïque, de 2,5 mg à 10 mg de co-enzyme Q10, de 2 mg à 8 mg de bêta-carotène, de 6 µg à 25 µg de vitamine D3, de 25 mg à 100 mg de vitamine E (source naturelle), de 45 mg à 180 mg de vitamine C, de 2 mg à 16 mg de thiamine, de 2 mg à 8 mg de riboflavine, de 9 mg à 36 mg de niacine, de 2 mg à 20 mg de vitamine B6, de 200 µg à 800 µg d'acide folique, de 5 µg à 18 µg de vitamine B12, de 75 µg à 300 µg de biotine, de 20 mg à 80 mg de vitamine B5 (acide pantothénique), de 50 mg à 200 mg, de 6 mg à 24 mg de fer, de 7,5 mg à 30 mg de zinc, de 0,5 mg à 2 mg de cuivre, de 0,25 mg à 1 mg de manganèse, de 50 µg à 200 µg de sélénium (exempt de levure), de 25 µg à 100 µg de chrome, de 100 µg à 400 µg d'iode (issue d'un extrait de varech purifié), de 25 mg à 100 mg de silicium, de 10 mg à 40 mg de L-cystine.

6. Utilisation selon la revendication 4, **caractérisée en ce que** les dosages suivants sont utilisés : 200 mg de collagène bio-marin (Cartidea), 15 mg d'extrait de pépins de raisin (95% de proanthocyanidines), 20 mg d'extrait de thé vert (extrait standardisé à 55% de polyphénols), 8 mg d'extrait d'écorce de pin (95% de proanthocyanidines), 10 mg d'extrait de lycopène à 5%, 50 mg d'huile de pépins de cassis, 40 mg d'acide alpha-lipoïque, 5 mg de co-enzyme Q10, 4 mg de bêta-carotène, 12,5 µg de vitamine D (D3, 500 UI), 50 mg de vitamine E (source naturelle), 90 mg de vitamine C, 8 mg de vitamine B1 (thiamine), 4 mg de vitamine B2 (riboflavine), 18 mg de vitamine B3 (niacine), 10 mg de vitamine B6, 400 µg d'acide folique, 9 µg de vitamine B12, 150 µg de biotine, 40 mg d'acide pantothénique, 100 mg de magnésium, 12 mg de fer, 15 mg de zinc, 1 mg de cuivre, 0,5 mg de manganèse, 100 µg de sélénium (exempt de levure), 50 µg de chrome, 200 µg d'iode (issue d'un extrait de varech purifié), 50 mg de silicium, 20 mg de L-cystine.

7. Utilisation selon l'une quelconque des revendications précédentes, dans laquelle la composition comporte un ou plusieurs véhicules ou excipients.

8. Utilisation selon l'une quelconque des revendications précédentes, dans laquelle la protection contre les effets de l'hiver sur la peau concerne spécifiquement l'amélioration de la viscoélasticité de la peau, l'augmentation de l'épaisseur de la peau, une protection contre la rugosité de la peau, et une protection contre des augmentations du microrelief cutané.
